# EUROPEAN PATENT SPECIFICATION

(11) **EP 0 642 326 B1**
(45) Date of publication and mention of the grant of the patent: **05.09.2001**
(21) Application number: 93914434.1
(22) Date of filing: 01.06.1993
(51) Int. Cl.: C08B 37/04, A61F 2/02, A01N 1/02, C12N 11/04, A61K 9/16

(54) **NON-FIBROGENIC, ALGINATE-COATED TRANSPLANTS, PROCESS OF MANUFACTURE AND METHOD OF USE THEREOF**
NICHT FASERIGE, MIT ALGINAT BESCHICHTETE TRANSPLANTATE SOWIE HERSTELLUNGS- UND ANWENDUNGSSVEFAHREN DAFÜR
TRANSPLANTS NON FIBROGENES REVETUS D'ALGINATE, PROCEDE DE PRODUCTION ET D'UTILISATION DE CES TRANSPLANTS

(30) Priority: 29.05.1992 US 891564
(43) Date of publication of application: 15.03.1995
(73) Proprietor: THE REGENTS OF THE UNIVERSITY OF CALIFORNIA, Oakland, California 94612-3550 (US); METABOLEX, INC., Hayward, CA 94545 (US)
(72) Inventor: DORIAN, Randel, E., Orinda, CA 94563 (US); COCHRUM, Kent, C., Davis, CA 95616 (US); VREELAND, Valerie, Berkely, CA 94705 (US)
(74) Representative: Thiel, Christian, Dr. Dipl.-Chem.
(86) International application number: PCT/US93/05461
(87) International publication number: WO 93/24077

(56) References cited:
- WO-A-89/04369
- US-A- 4 663 286
- US-A- 4 689 293
- ELECTROPHORESIS, vol. 13, no. 5, 22 May 1992 DE, pages 269-274, U. ZIMMERMANN ET AL. 'Production of mitogen-contamination free alginates with variable ratios of mannuronic acid to guluronic acid by free flow electrophoresis'

## Description

### BACKGROUND OF THE INVENTION

### Field of the Invention

This invention is directed to the field of medical transplants of cells and tissues, the manufacture of such transplants, and their use. In particular, this invention is directed to the coating of transplants with a novel, highly protective coating of alginate free of transplantation-impairing amounts of impurities, the coated tissues formed by this coating process and the transplants made using these products.

### Discussion of the Background

Traditional medical treatments for functional deficiencies of secretory and other biological organs have focused on replacing identified normal products of the deficient organ with natural or synthetic pharmaceutical compositions. For example, for treating insulin-dependent diabetes mellitus, also known as type I or juvenile onset diabetes, the normal secretion of insulin by the islets of Langerhans in the pancreas must be replaced since functional islets are no longer present in the pancreas. This pancreatic function is emulated by administering insulin, titrating the injections in response to blood glucose level measurements. At best, insulin production and secretion of the islets is imperfectly approximated and normally is poorly approximated.

Organ replacement has also been used. This has generally required continuous use of immunosuppressive agents to prevent immunological rejection of the organ, depriving the patient of the full protective function of the immune system against disease. This approach has provided permanent relief only for a limited group of organs. Attempts to transplant organ tissues into genetically dissimilar hosts without immunosuppression have been generally defeated by the immune system of the host. Prior to this invention, the application of effective protective barrier coatings to isolate the transplant tissues from the host immune system did not prove to be medically practical for a number of reasons. The coating materials were incompatible with the host system or otherwise unsuitable. The previously developed encapsulation or coating processes did not yield reproducible coatings having the desired porosity and thickness required for the transplanted tissue to have a long and effective functioning life in the host.

To protect transplants from destruction by the immune response of the host animal, various attempts have been made to create a protective barrier between the transplant tissue or cells and the immunological components of the host's system. T.M.S. Chang (Chang, T.M.S., *Science* 146: 524-525 (1964) described the microencapsulation of erythrocyte hemolysate and urease in semi-permeable polyamide membranes. These microcapsules did not survive for long when injected into the blood stream. Mosback et al and Chang et al. described the preparation of semi-permeable microencapsulated microbial cells and viable red blood cells, the latter article mentioning the possibility of using injections of encapsulated cells for organ replacement therapy. (K. Mosbach et al, *Acta Chem.Scand.* 20:2807-2812 (1966); Chang, T.M.S. et al, *Can.J.Psysiol.* and *Pharmacol.* 44:115-128 (1966)).

Viable tissue and cells have been immobilized in alginate droplets coated with polylysine by Lim et al. (F. Lim et al, *J. Pharm. Sci.,* 70:351-354 (1981)). An attempt to use these coated droplets to correct the diabetic state of diabetic animals was reported by Lim et al, (Lim et al, *Science* 210:908-909 (1981)). U.S. Patent Nos. 4,251,387, 4,324,683, 4,352,883, 4,407,957, 4,663,286, and 4,803,168 relate to this research. These products, however, have not been successful for the long term correction of the diabetic state of animals, and they have not proven suitable for transplanting tissues such as pancreatic islets in humans.

Substantial additional efforts made by Goosen et al. to develop transplants encapsulated in calcium alginate droplets reacted with polylysine were also unsuccessful in providing protected transplants suitable for transplantation. (U.S. Patent Nos. 4,673,566, 4,689,293, 4,789,550, 4,806,355, 4,789,550, for example).

Lim et al. reported the prolonged reversal of the diabetic state of NOD mice with xenografts of microencapsulated rat islets, using alginate-polylysine capsules. (Lim et al., Diabetes 40:1511-1516 (19 )).

U.S. Patent 4,744,933 describes encapsulating solutions containing biologically active materials in an outer membrane of inter-reacted alginate and polyamino acid.

U.S. Patent 4,696,286 describes a method for coating transplants suitable for transplantation into genetically dissimilar individuals by coating the transplant with a surface-conforming bonding bridge of a multifunctional material that binds chemically to a surface component of the transplant followed by a semipermeable, biologically compatible layer of a polymer that binds chemically to the bonding bridge layer.

Hackel et al and Kerstan et al report the use of calcium alginates for immobilization of microbial cells and enzymes. (Hackel et al., *J. Appl. Microbiol.* 1:291-296 (1975); Kerstan, M. et al, *Biotech. and Bioeng.*, 19:387-397 (1977)). Nigam et al. and publications cited therein describe methods for coating living cells in an outer membrane of calcium alginate by dropping a cell-containing calcium solution into an alginate solution and further incubating the capsules in a calcium solution. (Nigam et al, *Biotech. Tech*., 2:271-276 (1988))

Plunkett et al. describe an angiogenesis model using tumor cells entrapped in alginate beads. (Plunkett et al, *Lab. Invest.,* 90: 6204-6205 (1990)). A spray of sodium alginate-cell solution droplets was contacted with aqueous calcium chloride solution to form calcium alginate beads. Pump speed and air pressure were used to control the droplet size in the spraying process.

Calcium alginate coated implants, however, have not been previously considered suitable for use in transplanting tissues because the coated transplants did not survive in the host systems. Alginates in the form obtained from seaweeds are mixed polymers of guluronate and mannuronate containing various levels of other materials.

The gelation of calcium alginate is primarily caused by calcium ion bonding with the guluronic acid moieties of high guluronate polymers, which have higher porosity and provide higher levels of cross-linkage resulting in a strong protective barrier for the transplants. Skjak-Braek, alternatively, discloses the use of mannuronan C-5 epimerase, an enzyme capable of converting D-mannuronic acid residues which are already present an the alginate polymer into L-guluronic acid. (G. Skjak-Braek, *Biochem. Soc. Trans.:* 20-26 (1992)).

Although alginates have been applied to coating food and pharmaceutical products, the use of alginates for coating living cells to produce a non-immunogenic coating has heretofore not: been achieved. Those working in this field have recognized that it is necessary to purify alginates for cell coating applications. For example, filtration has been suggested as a means to remove proteins and polyphenols. However, even the purified alginates themselves are immunogenic. Soon-Shiong et al. have observed fibrous overgrowth of implanted alginate microcapsules in large animals. (Soon-Shiong et al, *Trans. Proc*., 23 :758-9, 1991)). This study found that commercial alginates are often contaminated with polyphenols and other immunogenic materials. However, even when purified, commercial alginates having high mannuronic acid content remained immunogenic and capable of activating macrophages in vivo and producing fibrotic overgrowth. Similarly, Espevik et al. suggest that all alginates are inherently fibrogenic. (Espevik et al, *Cell Trans.* 1:165 (1992)). T. Zekorn, *Cell Trans.* 1:176 (1992)). Others have reported that high mannuronate alginates stimulate human monocytes to produce cytokines (M. Otterlei, et al., J. Immunother. 10:286-291 (1991)), or enhance macrophage migration (M. Fujihara, and T. Nagumo, *Carbohydrate Research* 243:211-216 (1993)), suggesting why they are not biocompatible. Thus, it appears to be the consensus in the prior art that alginate, at least: alginate with a significant fraction of mannuronate, stimulates cytokine production, macrophage migration, and is inherently fibrogenic. Despite recognition in this field that purified alginates might be used for coating cells, long term non-immunogenic coatings have up to this time not been achieved.

Compositions of alginates and methods for purifying and fractionating alginates have been generally described (Haug, A., *Composition and Properties of Alginates: Report No.* 30, Norsk Institutt for Tang-og Tareforskning (Norwegian Institute of Seaweed Research) (1964); Haug, A. *Acta Chem. Scand.* 13:601-603 (1959); Haug et al, *Acta Chem. Scand.* 19:1221-1226 (1965); Haug et al, *Acta Chem. Scand.* 21:691-704 (1967); Smidrød et al, *Acta Chem. Scand.* 22:1989-1997 (1968); and Skjåak-Bræk et al, *Biotech. and* Bioeng. 33:90-94 (1989)). Correlations between the chemical and physical properties of alginate gel beads have also been reported (Martinsen et al, *Biotech. and Eng.* 33:70-89 (1989)).

Alginate coatings having a thickness greater than 200 *µ*m have been reported to lack the permeability required for permitting the flow of nutrients and cell products through the coating in amounts sufficient for long term viability of the coated transplant while implanted in the host system. (Chicheportiche et al, Horm. *Met. Res. Suppl.* 26:209-213 (1990)).

A need, therfore, continues to exist for novel coated transplants which are substantially not rejected by the host immune system.

Commercial alginates of variable homopolymeric regions of beta-D-mannuronic acid and alpha-L-glucoronic acid are potent stimulators of macrophages and lymphocytes. Mitogenic activity of commercial alginates was linked to fractions that could be separated by electrophoresis : Zimmermann et al. Electrophoresis 13:269 (1992). WO-A-89 04 369 refers to the purification of alginates by treatment with activated charcoal.

### SUMMARY OF THE INVENTION

This invention relates to a purified non-fibrogenic alginate composition suitable for coating of physiologically active, tissue or cells for transplantation which are physiologically acceptable to the host, and which effectively provide prolonged protection of the transplanted tissue cells from destruction by the host immune system, the composition being defined in claim 1.

Tissue cells coated with this composition of the invention are enclosed in a coating capsule having a thickness permitting the diffusion to the transplanted cells or tissue of the amounts of nutrients and other substances required for their health, long life and effective function after transplantation, and a permeability allowing the effective diffusion and release of transplanted tissue or cell products into the host system.

This invention further relates to a divalent metal ion alginate gel, as defined in claim 9, which is physiologically acceptable, non-fibrogenic and non-toxic to the host, and which can be used tc provide a coating having the characteristics describes above.

Still part of this invention is a manufacturing process for effectively coating transplant tissue and other biological substances with a complete barrier coating which is physiologically acceptable, non-fibrogenic and non-toxic to the host and which provides a complete barrier coating with a controlled thickness and permeability to intermediate size proteins, as defined in claim 13.

The coating is substantially free from fibrogenic concentrations of fucose, polyphenol and protein. The amount of fucose groups in the coating is, in general, less than 0.2 micrograms per milligram of sodium alginate (even less than 0.02 wt%) and the amount of polyphenol groups is, in general, less than 2.0 micrograms of tannin-equivalents per milligram of sodium alginate (even less than 0.2 wt.%).

### DETAILED DESCRIPTION OF THE PREFERRED EMBODIMENTS

This invention arose from a desire by the inventors to improve on prior art coatings for tissue transplants, and to provide a composition which, upon being coated onto a tissue to provide a tissue transplant, substantially fails to evoke detrimental reactions upon implantation into a host, such as fibrogenesis and/or host rejection.

The present inventors have discovered that the drawbacks of prior art transplants were the result of several factors, such as certain natural materials present in commercially available alginate preparations being fibrogenic to the host tissue surrounding a transplant. This fibrogenicity, in turn, leads to the encapsulation of the transplant in an impervious, poorly vascularized layer of scar tissue which causes transplant necrosis and disfunction. The present inventors also found that the prior art had failed to remove from the alignates sufficient amounts of substances containing fucose, polyphenols and protein which are instrumental in the developing fibrosis. Although prior to the present invention, transplanted pancreatic cells were said to produce insuline, inspection of the transplanted coated cells always revealed the presence of significant fibrosis. On the contrary, transplants of cells coated with the purified alginates of this invention substantially fail to produce fibrosis.

The present inventors have developed and provide herein efficient procedures for the mass production of transplants having alginate coatings with a thickness of less than about 200 *µ*m, and have found that they can be used upon transplantation to restore, e.g., pancreatic islet function indefinitely.

Prior to this invention, reacting the outer surface of alginate coatings with polylysine was reported to be necessary for alginate coated transplants. The present inventors have developed fully operable, non-fibrinogenic coatings with perfected permeabilities which do not require a secondary reaction of the outer coating with polylysine.

One aspect of this invention, thus, is a novel, alginate composition which is substantially non-fibrogenic. This composition is provided substantially free from substances which may impair the acceptance by the host of transplants coated with divalent metal ion alginate gel products thereof.

In the present invention, an initial fibrogenic alginate preparation is purified to yield a non-fibrogenic alginate suitable for coating cells with a non-fibrogenic coating. Suitable initial alginate preparations are preferably obtained by isolating alginates from brown algae and are commercially available. In this invention, the initial alginate preparation is contacted with a divalent metal ion-chelating agent to remove divalent metal ions, and then contacted with high surface area, bleached, activated carbon. The carbon adsorbs polyphenols together with associated protein and fucose moieties. Compositions containing protein, polyphenols, and fucose moieties are collectively referred to in the art as "fucans". After treatment with carbon, the alginate is precipitated from solution, washed and then filtered to remove additional impurities and to provide the non-fibrogenic alginate of the present invention.

The term "non-fibrogenic" as used herein means a composition which, when used to produce an implant having a diameter of 500 microns or less, does not induce bioisolation and resulting tissue death of the implant by the immune system of a host through the process of fibrosis and macrophage overgrowth for at least 60 days, preferably at least 6 months, more preferably at least 12 months, and most preferably at least 18 months after interperitoneal injection of an alginate coated implant into a host BALB/c mouse or mongrel dog. Bio-isolation and tissue death can be determined, for example, by monitoring insulin production from a pancreatic islet tissue implant or by monitoring the maintenance of euglycemic in a diabetic host. The non-fibrogenic alginate of the invention is suitable for coating tissues or single cells using known coating technology.

The coated transplant tissues and cellls obtained according to this invention are effective for implantation into a host animal by simple injection through a hypodermic needle having a needle diameter sufficient to permit passage of a suspension of coated cells therethrough without damaging the tissue coating. For implantation, the coated transplant tissues are formulated as pharmaceutical compositions together with a pharmaceutically acceptable carrier. Such compositions should contain the highest number of coated transplant capsules which can be effectively injected into a mammal in order to provide a sufficient number of transplant capsules during transplantation.

The term "transplant", as used herein, is defined to include all living tissues, cells, and biologically active substances intended to be implanted into the body of a host animal and the act of implanting these tissues and cells. These tissues and cells include, without limitation, tissue and cells removed from a donor animal, tissue and cells obtained by incubation or cultivation of donor tissues and cells, cells obtained from viable cell lines, biologically active products of cells and tissues. Any type of tissue or cells for which transplantation is desired can be coated and transplanted according to this invention. The most important tissues for transplants are secretory organ tissues, where transplantation from a donor organ to a host animal is desired to at least partially replicate the donor organ's action in the host system. Preferred donor tissues are pancreatic islets, hepatic cells, neural cells, renal cortex cells, vascular endothelial cells, thyroid cells, adrenal cells, thymic cells and ovarian cells. However, other types of cells may also be utilized.

The process of this invention is described hereinafter for the preparation and transplantation of pancreatic islets and islet cells by way of example, for purposes of clarity of explanation and not by way of limitation. This process can be equally well applied to other organ tissues as will be readily apparent to a person skilled in the art, with conventional and obvious modifications as required to accommodate any uniquely different requirements of the different tissues. Applications of the process to all tissues and cells suitable for transplantation are intended to be within the scope of this invention.

Isolated pancreatic islets (or other cells or tissues suitable for transplantation) may be prepared by conventional procedures to separate them from extraneous tissue and donor substances.

To prepare the non-fibrogenic alginate of the invention, an initial alginate preparation is dissolved in water or buffer, preferably containing a divalent metal ion-chelating compound such ethylenediamine tetraacetic acid (EDTA), among others, and then contacted with a high surface area, activated carbon to remove any fucans and other contaminants present by adsorption. About fifty grams of alginate is usually dissolved in 1 to 10, and more preferably 3 to 8 liters, of water. Suitable activated carbon includes any high surface area activated carbon having a particle size of about 100 mesh or finer. Preferably, very fine activated carbon powder having a surface area of at least about 1,000, and preferably at least about 1,500 m²/g, may be used. Suitable activated carbon is commercially available.

The activated carbon is preferably bleached to oxidize and remove organic contaminants prior to use. The activated carbon may be bleached using a known bleaching agent such as sodium hypochlorite, and the like. The carbon may be bleached by stirring it with a dilute solution of the bleach for a time sufficient to remove any contaminants from the surface of the carbon. Generally, stirring the activated carbon with a 0.005 to 0.50 M, and more preferably a 0.08 to 0.10 M, sodium hypochlorite solution for 5 to 30 minutes, and preferably 10 to 20 minutes, which is is sufficient to oxidize the activated carbon. After oxidation, the activated carbon may be removed from the dilute bleach solution by centrifugation or filtration, washed with water or ethanol, and dried. The ratio (w/w) of initial alginate to activated carbon is usually 1:1 to 1:20, and more preferably 1:2 to 1:8. Obviously, the amount of activated carbon may be adjusted as necessary to insure the removal of sufficient contaminants to achieve the minimum amounts permitted by this invention.

The alginate solution and bleached carbon may be contacted by simple mixing, shaking or rolling, and the bleached carbon may then be removed by conventional centrifugation and filtration. Preferably, the filtration is conducted using sequentially finer sub-micron filters.

A dilute monovalent cation salt solution is then added to the filtered alginate solution in an amount sufficient to exchange metal ion binding sites on the alginate. Any soluble monovalent cation salt may be used. Sodium or potassium chloride solutions of 0.01 to 1.0 M are, however, preferred.

The alginate may then be precipitated from the resulting solution by addition of ethanol with stirring. Generally, the ratio (v/v) of ethanol to alginate solution is 0.25 to 2.0, and preferably 0.5 to 1.5. The precipitated alginate may then be recovered by filtration, washed with ethanol and dried to remove traces of ethanol.

The alginate obtained as described above is directly suitable for coating a transplant tissue or cells without the use of additional coating compounds such as homopoly(amino acids), e.g. polylysine or polyaspartic acid. However, it is sometimes desirable to chemically modify the properties of the alginate to tailor the alginate coating to specific uses. Important properties of the alginate coating materials include, for example, a well defined and controlled pore size, coating thickness, viscosity of coating solutions, mechanical strength, etc.

The average molecular weight and overall mannuronate to guluronate molar ratios are initially substantially determined by the origin of the material, but may be further adjusted by physical and chemical methods, as well. The molecular weights may be reduced, for example, by partial acid hydrolysis, thermal degradation or sonication. High molecular weights may be obtained by controlled precipitation methods with the concomitant alteration of the alginate composition, or by dialysis, molecular filtration, or gel exclusion chromatography. The mannuronate to guluronate ratio and sequence distrubtion may be increased or decreased by selective precipitation or solubilization by mono- and di-valent metal cations, organic solvents or acids. Adjustment of these characteristics provide optimum results with different tissue transplants.

The concentration of an alginate in solution is a function of the physical properties of the alginate. At very low concentrations, the coating morphology is poor resulting in ineffective coatings. At very high concentrations, the viscosity is too high to form good coatings. The molecular weight (kilodaltons) of the alginate is in the range of 6 to 120. If the initial molecular weights are high, they may be reduced by mild acid hydrolysis of the alginate. The initial or purified alginate may be dissolved in a dilute acid solution and gently heated until the desired molecular weight is obtained. Dilute solutions of mineral acids such as HCl, etc., having a concentration of 0.1 to 0.5 M may be used. The degree of hydrolysis may be controlled by monitoring the molecular weight of the alginate and neutralizing the hydrolysis reaction when the desired molecular weight is obtained. Obviously, higher acid concentrations result in faster hydrolysis. Alternatively, a few initial test reactions are usually sufficient to determine appropriate hydrolysis conditions.

The porosity and mechanical strength of the coating are also a function of the relative amounts of mannuronate (M) ' and guluronate (G) in the alginate polymers. Preferably, the amount of M, calculated as M/ (M+G), in the alginate is in the range of 0. 25 to 0.75. The relative amount of M and G in the alginate may be adjusted by dissolving precipitated alginate in a dilute (e.g., 0.05 to 0.50 M) potassium chloride solution to redissolve G-rich fractions while M-rich fractions are left in the precipitate. The insoluble material may be collected by centrifugation. The redissolved G-rich material may then be reprecipitated by addition of ethanol. By repeating this process, any desired relative proportion of M and G in the alginate may be obtained.

Homopolymeric alginate sequences (polymannuronate and polyguluronate) are generally acid-insoluble, whereas alternating mannuronate-guluronate sequences are for the most part acid-soluble. By extracting the alginate with an acid solution of pH 1.5 to 2.5, and preferably pH about 2.0, it is possible to selectively solubilize homopolymeric-rich alginates. Additionally, M-rich alginates are preferentially solubilized relative to G-rich alginates. The treatment of an alginate with an acidic solution, therefore, precipitates G-rich alginates preferentially leaving M-rich alginates in solution. The separation of the precipitate from the solution thus provides both G-rich and M-rich alginate fractions. The G-rich alginates present in the solution may be precipitated by addition of calcium ions or ethanol. Alternatively, the G-rich alginates may be obtained by precipitation of the G-rich fractions with calcium ions while leaving the M-rich fractions in solution. After separation of the precipitate from the solution, the M-rich alginate fraction may be precipitated from solution by addition of acid or ethanol. The proportions of acid and/or calcium precipitated materials may be controlled by adjusting the pH and the calcium concentration, respectively.

It is also possible to obtain specific relative amounts of M and G in the alginate coating by mixing different M-rich fractions and G-rich fractions obtained as described above. By sequentially adding small portions of G-rich material to M-rich material, the amount of G in the overall alginate composition may be gradually increased, thereby increasing the number of divalent metal ion binding sites in the overall coating, increasing the structural rigidity of the coating, and producing larger pore sizes. For any particular mixture of M-rich and G-rich fractions, the relative amount of M or G in the alginate may be readily determined by NMR spectroscopy. ¹³C-NMR spectroscopy is a rapid and inexpensive method for determining the M/G ratio and disaccharide sequence frequencies but 'H-NMR may also be used to determine M/G ratios. The average molecular weight and porosity of the alginate coating may be adjusted by mixing M-rich and G-rich fractions in different proprotions.

The alginates of the present invention are non-fibrogenic, having a hydrolyzable fucose sugar content of 0.2 or less micrograms per milligram of sodium alginate (even 0.02 wt% or less), preferably less than 0.1 micrograms per milligram sodium alginate (even 0.01 wt.% or less), and even more preferably less than 0.05 micrograms per milligram sodium alginate (even 0.005 wt.% or less) . The fucose sugar levels in the alginates may be determined by conventional neutral sugar analysis, such as is described in Example 4 below.

The alginates of the present invention contain a non-fibrogenic amount of polyphenols, such as tannins or phloroglucinol. To determine the level of polyphenols in the alginate of the present invention, the inventors developed a novel assay based on a standard method for the measurement of tannin levels in water. (See, *Hach's Water Analysis Handbook,* Second Edition (1992), Method 8193, adapted from M.B. Kloster, *Journal* of *the American Water Works Association* 66:44 (1974)). Tannins are polyphenols and the novel alginate assay of this invention for polyphenols gives the polyphenol content in terms of tannic acid or "tannin-equivalents" based upon standard tannic acid solutions of known concentration.

In the present assay for polyphenols in alginates, the alginate samples are prepared at a concentration of about 1 wt.% in water. An aliquot of the alginate sample is placed into a test tube and an equal volume of water is placed into a control test tube. Similar volumes of a sodium carbonate solution and of TANNIVER® 3 reagent (sodium tungstate, phosphoric acid, hydrochloric acid, anhydrous sodium molybdate, lithium sulfate, and water plus 1% of other reagents) are added to both the sample the and control, the solutions are then mixed and incubated at room temperature for about 30 minutes. The absorbance of the sample and control is then measured at about 700 nm.

A suitable standard curve for tannic acid may be prepared using tannic acid standard concentrations varying over a range of about 0.1 to 10 *µ*g/ml. After correcting the absorbance of any sample by subtracting the absorbance of the control, the absorbance of the alginate sample may be plotted against a standard curve prepared from the standard tannic acid solutions to determine the number of micrograms of tannic acid present per milligram of alginate sample or the micrograms of "tannin-equivalents" per milligram of alginate. When tested with the polyphenol/tannin assay described above, the alginates of the present invention are shown to contain about 2.0 micrograms or less of tannin-equivalents per milligram of sodium alginate (even 0.2 wt.% or less) . Preferably, the alginates contain about 1.0 micrograms of tannin-equivalents or less (even 0.1 wt.% or less), and more preferably about 0.75 micrograms of tannin equivalents or less (even 0.075 wt.% or less) per milligram of sodium alginate.

The method of the present invention allows the preparation of an alginate gel coating suitable as a protective barrier which excludes immunologically effective concentrations of agents of the host immune system from contracting the tissue and having the permeability required to permit sufficient diffusion of nutrients and other substances so that they become in contact with the transplants required for their long life and viability.

The viscosity of coating solutions of the alginates having a concentration of 0.7 to 2.5 weight percent alginate should have a viscosity of 10 to 250 centipoises and preferably 20 to 100 centipoises, at 25°C.

In the first step of the process of this invention, isolated pancreatic islets (or other cells or tissue) may be washed with isotonic saline and suspended in a solution of purified alginate. Optionally, but not necessarily, the washed cells may be pretreated with an aqueous solution of poly-L-lysine to increase the bonding of the cells with the alginate, and then rinsed with saline.

The cell suspension in the alginate is formed into droplets, and the droplets are contacted with a divalent metal, preferably an alkaline earth metal, salt solution to gel the alginate. The droplets may be formed by any conventional procedure. For example, alginate droplets may be formed by emulsifying a solution of sodium alginate containing cellular material to form droplets of sodium alginate and cells, and gelling the droplets with calcium chloride (U.S. Patent 4,352,883). Alginate droplets may also be formed with a syringe and pump to force the droplets from a needle, using a laminar flow air knife to separate the droplets from the tip, the droplets being gelled by collection in a calcium chloride solution (U.S. Patent 4,407,957). Alginate droplets may also be formed by expulsion from a hypodermic needle, and then allowing the droplets to fall into a calcium chloride solution (Nigam et al, *Biotechnology Techniques* 2:271-276 (1988)). In addition, droplets may also be injected into a concurrently flowing stream containing calcium chloride (U.S. Patent 3,962,383). Spraying alginate solutions through a spray nozzle to form a mist of droplets which was collected in a calcium chloride solution may also be utilized (Plunkett et al, *Laboratory Investigation.* 62:510-517 (1990)).

Alginate droplets may also be formed using a combination of a needle and a pulsed electrical electrostatic voltage to form uniform droplets (U.S. Patent 4,789,550 to Hommell et al.). The alginate solution may be forced through a needle tip to form a droplet, and the droplet pulled from the needle by a changing electrostatic field between the needle tip and a calcium chloride solution placed below the needle tip. The droplet receives a charge of one polarity from the needle, which is opposite the charge in the calcium chloride solution. When the voltage difference between the droplet and the oppositely charged calcium chloride solution reaches a threshold value at which the attraction by the solution on the droplet exceeds the force of interfacial tension holding the droplet on the needle tip, the droplet is pulled free to fall into the calcium chloride solution. The electrostatic field may be fluctuated using a square wave form to create a succession of voltages crossing the threshold value, thus producing a continuous series of droplets, one per square wave cycle. This process does not appear to provide the small droplets and thin coatings required for effective transplantation.

A preferred drop forming and gelling procedure for use herein is described in U.S. patent application Serial No. 07/890,982, filed May 29, 1992.

The tissue containing viable, physiologically active, tissue cells such as pancreatic islet cells is provided a coating having a thickness of 10 to 200 *µ*m. The coating comprises a divalent metal alginate gel, preferably a calcium alginate, magnesium alginate, or mixtures thereof, the metal alginate having been formed from an alginate free from impurities which would impair the viability and long life of tissue transplants coated with the alginate. The coating capsule has preferably a sufficiently low permeability and a sufficiently large thickness to protect the tissue cells from host immunological agents after transplantation, the coating also being sufficiently permeable and thin to permit the diffusion of sufficient cell nutrients and cell products through the coating required for cell viability. The coating preferably has a thickness of at least about 10 *µ*m and less than about 50 *µ* m. Most preferably, a single cells or 1-2 cell(s) are present within a single capsule. If desired, a plurality of alginate coatings may be applied over the cells to produce a multilayered capsule.

If a further reduction of molecular permeability is desired or a reduction of swelling of the alginate coating is required, the alginate gel coating may optionally, but not necessarily, be cross-linked with poly-L-lysine or other physiologically acceptable, non-toxic polycation polymer by immersing the coated tissue in a solution of the polycation polymer. The reduction in permeability is a function of the degree of polymerization of the polycation, the reaction of the polycation with the alginate coating, the concentration of the polycation in solution, and the incubation time. The selection of the optimum polycation and the reaction conditions is conventional and fully within the skill of the art. The reaction may be terminated by depletion of the polymer from the solution or by dilution of washing.

Polylysine, and polycations in general, induce fibrosis and a further treatment of the alginate-polycation complex is desirable to improve biocompatibility of the coated product. The immersion of the polycation reacted products in a solution of sodium alginate to react free epsilon-amino groups at the coating surface leads to an ion-exchange reaction in which the metal alginate core is partially or completely solubilized by depletion of the divalent metal cations therefrom. However, if trace amounts of soluble alginate remain after such treatment, a slow diffusion of the liquified materials from the capsule may lead to a fibrotic reaction, particularly when the alginate is known to induce fibrosis in its soluble form. If the alginate treated products are treated with a divalent metal ion before complete solubilization of the alginates and their removal from the core, the metal ion may react with the sodium alginate migrating outwardly across the coating layer, thereby compromising the distinctness of the membrane and increasing the likelihood of fibroblastic adhesion to the coating surface.

Therefore, if alginate is to be used in the outer coating, the reaction should be carried out to completely dissolve the core gel either by ion-exchange or chelation of the calcium ion, for example with sodium citrate or EDTA. The product may then be washed exhaustively with several changes of wash medium or by percolation, allowing ample time for the soluble alginate to diffuse entirely out of the coating. As the alginate diffuses outwardly through the coating, free residual amino groups of the polycation may react therewith.

Preferably, a negative charge is applied to the polycation-complexed, alginate-coated tissue transplants by reacting them with polyaspartic acid. Because of its lower binding affinity, polyaspartic acid is less likely to complex and deplete metal ions from the primary alginate gel coating. It reacts with the polycation without dissolving the primary alginate coating. The use of polyaspartic acid as the final reactant provides several advantages over the use of an alginate final complex. It provides greater mechanical strength, smaller coated product diameter, and low permeability due to the additional cross-linking and volume reduction of the condensed coating.

This invention is further illustrated by the following specific but non-limiting examples. Percents are given in weight percents and temperature in degrees Centigrade, unless otherwise specified. In these examples, procedures which have been reduced to practice in the laboratory are presented in the past tense, and procedures which are constructively reduced to practice in this application are presented in the present tense.

### EXAMPLES

### Example 1-High Mannuronate Alginate Preparation

50 g low viscosity sodium alginate (LV Alignate, KELCO Div. of Merck & Co.) isolated from Macrocystis pyrifera were dissolved in 5 liters of water and filtered through a 50 micron mesh to remove particulates. 18.6 g disodium EDTA were added to the solution and dissolved. The solution was mixed on a roller mill with 200 g hypochlorite-bleached activiated carbon (Mallinckrodt activated carbon powder) for 30 minutes to remove organic contaminants such as polyphenols and fucose sugar residues. Activiated carbon was then removed by centrifugation for 30 minutes. The resulting solution was sequentially filtered through filter paper, a 0.45 micron filter, a 0.22 micron filter and a 0.1 micron filter. 30 g. sodium chloride were then added to the filtered solution and dissolved by rolling on a roller mill. The alignate was precipitated from solution by the addition of 5 1 neat ethanol. The sample was centrifuged for 30 minutes to obtain an alginate pellet and the alginate pellet was suspended in ethanol and then teased apart with tweezers to insure complete washing of the sample. Excess ethanol was removed by squeezing and pressing the precipitate. The resulting precipitate was dried in an oven, under vacuum, at 60°C.

### Example 2-High Guluronate Alginate Preparation

80 g protan alginate were dissolved in 89 1 water by rolling on a roller mill. The solution was filtered through a 50 micron mesh to remove particles, and then mixed on a roller mill with 320 g of bleached, activiated carbon with continued mixing for 30 minutes. The activated carbon was then removed by centrifugation for 30 minutes. The resulting solution was sequentially filtered through filter paper, a 0.45 micron filter, a 0.22 micron filter and a 0.1 micron filter. 163 g magnesium chloride were then added to the solution and dissolved by rolling on a roller mill. 210 ml of a 1.7% calcium chloride solution were then added and mixed by rolling on a roller mill for 30 minutes. The resulting solution was centrifuged for 30 minutes to produce an alginate pellet. The alginate pellet was dissolved in 3.0 liters of 0.1M EDTA, pH 7.0 by rolling on a roller mill. The pH of the solution was adjusted to pH 7.0, as needed. 20 g sodium chloride were then added to this solution and dissolved.

Alginate was percipated from the solution by the addition of 5 1 of neat ethanol, followed by centrifugation for 30 minutes to obtain an alginate pellet. The alginate pellet was then suspended in ethanol and tweezed apart with tweezers to insure complete washing of the sample. Excess ethanol was then removed by squeezing and pressing the precipitate. The alginate precipitate was then dried in an oven, under vacuum, at 60°C.

### Example 3-Fucose and Tannin Equivalent Content Dertermination

Standard tannic acid solutions were prepared by diluting a freshly prepared tannic acid solution (0.1 mg/ml) to obtain standard solutions having a concentration of 10.0, 5.0, 3.0, 1.0, 0.4 and 0.1 *µ*g/ml. A tannic acid standard curve was then prepared by plotting the concentration of tannic acid against the absorbance of each sample read at 700 nm in a spectrophotometer.

Sample solutions of the alginates of Examples 1 and 2 were prepared at 1 wt.%. A 2 ml aliquot of each sample was placed into a 5 ml test tube. Two ml of water were placed into a test tube as a control. Sodium carbonate solution (0.4 ml of Hach Cat. 675-49) was then added to each tube, including the control. TANNIVER® 3 reagent (0.04 ml) was added to each tube and thoroughly mixed. The tubes were then incubated at room temperature for 30 minutes. The sample was transferred to a quartz cuvette, and the absorbance read at 700 nm in the spectrophotometer. After correcting for the absorbance of the sample blank cuvette, the absorbance of each alginate solutions was plotted against the tannic acid standard curve to determine the number of micrograms of tannic acid equivalents per milligram of sodium alginate sample. The alginate of Example 1 was found to contain 1.0 micrograms of tannic acid equivalents per milligram of sodium alginate (0.1 wt.%). The alginate of Example 2 was found to contain 0.7 micrograms of tannic acid equivalents per milligram of sodium alginate (0.07 wt.%).

For comparison, the initial alginates used to prepare the alginates of Example 1 and Example 2 were assayed for polyphenol content using the polyphenol/tannic acid assay described above. Each of the initial alginates used to prepare the purified alginates of Examples 1 and 2 was found to contain about 2.0 micrograms of tannic acid equivalents per milligram of alginate. The purification process of Example 1 provides a reduction in tannic acid equivalents greater than about 90% and the process of Example 2 provides a reduction in tannic acid equivalents greater than about 90% relative to the initial alginate compositions. The process of the present invention is effective in substantially reducing the level of polyphenols in the initial alginates.

### Example 4-Fucose Sugar Analysis

The content of fucose in alginate samples can be determined by gas chromatography-mass spectrometry (GC-MS) analysis. To prepare samples for GC-MS analysis, 1-3 mg of each sample was weighed and placed in a screw top (100 mm x 13 mm) test tube. H₂SO₄ (0.5 ml at IN) containing 50 *µ*g inositol as an internal standard was then added to each tube. Standard tubes containing 10.0, 1.0, 0.1 and 0.01 *µ*g fucose were then prepared in a similar manner.

All tubes were heated for 1 hour (or 3 hours) at 121°C. After heating, the tubes were cooled and a stoichiometric quantity of barium chloride was added. The neutralized tubes were centrifuged (10 min, 1500 x g) to remove the barium sulfate produced. Water remaining in the samples was evaporated under an air stream.

The samples were then reduced with sodium borohydride (1 mg/ml NaBH₄ in IN NH₄OH, 0.5 ml solution, 1 hour at room temperature). After reduction, excess borohydride was decomposed by addition of 200 microliters of glacial acetic acid and evaporated under an air stream.

The reduced samples were acetylated using 200 microliters of acetic anhydride and 20 microliters of 1-methylimidazole (10 min at room temperature). The samples were then partitioned between 2 ml water and 2 ml methylene chloride. The water phase was removed and the remaining organic phase was evaporated. Fifty microliters of acetone were then added to each sample and an aliquot of the sample was injected into a 5890 HP gas chromatograph coupled to a 5970 HP mass selective detector. The GC separation was performed by a J & W DB-23 column (30 meters, 0.25 mm I.D.) using a temperature gradient running from 160 to 210°C at 3°/min. MS analysis was performed by comparison of retention times and of spectral comparisons with authentic standards.

The results of fucose analysis for the initial alginates and purified alginates of Examples 1 and 2 are shown in the table below.

**Table:**

| Fucose Analysis Results | |
|---|---|
| SAMPLE | FUCOSE (*µ*g fucose/mg sample) |
| Ex. 1 (initial) | 2.91 (0.291 wt.%) |
| Ex. 1 (purified) | <.01 (<0.001 wt.%) |
| Ex. 2 (initial) | 0.70 (0.07 wt.%) |
| Ex. 2 (purified) | 0.09 (0.009 wt.%) |

The fucose level in Example 1 (purified) is reduced by a factor of more than 300 relative to the fucose level in Example 1 (initial). The fucose level in alginate Example 2 (purified) is reduced by a factor of 8 relative to Example 2 (initial).

### Example 5-Preparation of Pancreatic Suspension Islet

Pancreatic islets isolated from rat were washed with isotonic saline, were suspended in an alginate solution prepared by dissolving the alginate prepared by the procedures of Example 1 and Example 2 at a concentration of 10,000 islets per ml in 1.9 wt.% purified alginate in 10 mM HEPES, 0.01 M sodium citrate, containing sufficient sodium chloride required for isoosmolarity (about 0.81 wt.%), the final solution having a viscosity of about 50 centipoises at 32°C. The islets had an approximate average diameter of 150 *µ*m. This procedure was repeated with dog islets.

### Example 6-Coating of Pancreatic Islets

Using a DC electrostatic voltage of 8 KV provided by a van de Graaff generator between needle tip and grounded 0.117 M aqueous calcium chloride solution at ambient temperature, a suspension of pancreatic islets (25 islets per *µ*L) prepared by the procedure of Example 5 was passed through a 20 gauge needle at a flow rate of approximately 200 *µ*l/min. The suspension emerged from the needle as a thin, attenuated stream which transformed into droplets, the droplets being collected in the calcium chloride solution. The droplets were gelled by reaction with the calcium ion in the solution. The calcium alginate coatings on the islets were smooth and uniform and had an approximate thickness of about 130 *µ*m. The total coated particle had an average diameter of about 360 *µ*m.

This process was repeated with dog islets prepared by the procedure of Example 5.

### Example 7-Pancreatic Islet Transplant into Diabetic Mice (IP)

Host Balb/C mice were rendered diabetic by IP injection of streptozocin (250 mg/kg) at 50 mg/mL in 0.1 M citrate buffer, pH 4.5 several days prior to transplant.

Coated dog islets of Langerhans prepared by the procedure of Example 6 were injected IP, 2000-3000 islets per mouse, into one group of mice. The mice became and remained euglycemic for over 72 weeks (18 months). Several mice returned to the diabetic state several weeks after implantation. These mice were sacrificed and the coated islets examined. The alginate-coated islets were found to be viable, free from fibrosis and free from macrophage overgrowth (only 2-10 macrophages per coated islet capsule).

Spheres formed from the same alginate (without cells) were injected IP into a control group of Balb/C mice. The mice were sacrificed at intervals for periods of a few days to several weeks. The alginate spheres were examined histologically and found to be free from fibrosis and substantially free from macrophage overgrowth.

Obviously, numerous modifications and variations of the present invention are possible in light of the above teachings. It is therefore to be understood that within the scope of the appended claims, the invention may be practiced otherwise than as specifically described herein.

## Claims

1. A purified non-fibrogenic alginate composition suitable for coating of functional tissue or cell transplants having substantially reduced amount of fucose, polyphenols and proteins,
wherein the non-fibrogenic alginate is purified to contain less than 0.02 wt.% of fucose and less than 0.2 wt.% of polyphenols expressed in terms of tannic acid;
wherein the molecular weight of the alginate is in the range of 6 to 120 kilodaltons;
wherein the alginate comprises a mixture of mannuronate and guluronate wherein the amount of mannuronate, calculated as a ratio of mannuronate/mannuronate + guluronate, in the alginate is in the weight range from 0.25 to 0.75;
wherein the alginate is non-fibrogenic when used for coating of cells; and
wherein said cells coated with said non-fibrogenic alginate implanted into a mouse substantially fail to induce fibrosis for at least 60 days after implantation.

2. The composition of claim 1 prepared by a purification process, and comprising steps:
(a) preparing an aqueous alginate solution comprising a mixture of mannuronate/mannuronate + guluronate in a weight ratio from 0.25 to 0.75 and a divalent metal ion-chelating agent;
(b) contacting said alginate solution with bleached activated carbon in an amount and for a time sufficient to adsorb fucose, polyphenols, proteins and other contaminants present in said alginate to produce a non-fibrogenic alginate and then removing said activated carbon from said alginate solution;
(c) adding ethanol to said alginate solution in an amount effective to precipitate said alginate from said alginate solution; and
(d) isolating said precipitated non-fibrogenic alginate composition.

3. The composition of Claim 2, wherein the precipitated non-fibrogenic alginate composition of step (d) is used for coating a viable, functional tissue or cells and gelled with divalent metal ions.

4. The composition of Claim 3, wherein the amount of fucose remaining in the composition is less than 0.01 wt.%.

5. The composition of Claim 3, wherein the amount of fucose remaining in the composition is less than 0.005 wt.%.

6. The composition of Claim 3, wherein the amount of polyphenol remaining in the composition is less than 0.1 wt.% in terms of tannic acid.

7. The composition of Claim 3, wherein the amount of polyphenol remaining in the composition is less than 0.075 wt.% in terms of tannic acid.

8. The composition of Claim 2, wherein in step (b) the ratio of alginate to activated carbon is from 1:1 to 1:20.

9. A non-fibrogenic divalent metal ion-alginate gel, comprising divalent metal ions and a non-fibrogenic alginate purified with activated high surface carbon having a molecular weight of 6 to 120 kD, a weight ratio of mannuronate/(mannuronate + guluronate) of 0.25 to 0.75, wherein the amount of fucose is less than 0.02 wt.% and the amount of polyphenol is less than 0.2 wt.% in terms of tannic acid, and wherein when a cell coated with said alginate gel is implanted into a BALB/C mouse it substantially fails to induce fibrosis for at least 60 days after implantation.

10. The alginate gel of Claim 9, wherein said gel fails to substantially cause fibrosis for at least 6 months after implantation.

11. The alginate gel of Claim 9, wherein said gel fails to substantially cause fibrosis for at least 12 months after implantation.

12. The alginate gel of Claim 9, wherein said gel fails to substantially cause fibrosis for at least 18 months after implantation.

13. A process for coating tissue with a non-fibrogenic alginate comprising:
(a) preparing an aqueous alginate solution comprising alginate having a molecular weight of 6 to 120 kD comprising a mixture of mannuronate/mannuronate + guluronate in a weight ratio from 0.25 to 0.75 and a divalent metal ion-chelating agent;
(b) contacting said alginate solution with bleached activated carbon in an amount and for a time sufficient to adsorb fucans present in said alginate to produce a non-fibrogenic alginate having substantially reduced amount of fucose polyphenols and proteins wherein the amount of fucose is reduced to less than 0.02 wt.% and the amount of polyphenol, expressed in terms of tannic acid to less than 0.2 wt.%;
(c) removing said bleached activated carbon from said alginate solution;
(d) adding ethanol to said alginate solution in an amount effective to precipitate said alginate from said alginate solution;
(e) isolating said precipitated alginate; and
(f) coating viable, physiologically active tissue with said precipitated non-fibrogenic alginate.

14. The process of Claim 13, wherein said bleached activated carbon is obtained by contacting activated charcoal having a particle size of 100 mesh or finer with a 0.005 to 0.50 M sodium hypochlorite solution for about 5 to 30 minutes.

15. The process of Claim 14, wherein the ratio of bleached activated carbon to alginate in said alginate solution is 1:1 to 1:20 (w:w).

16. The process of claim 15 wherein the non-fibrogenic alginate is used for preparation of an alginate gel capsule, comprising viable, physiologically active coated tissue or cells.

17. The process of Claim 16, wherein the coating has a thickness of at least 10 µm and less than 200 µm.

18. The process of Claim 16, wherein the tissue comprises at least one or more cell types selected from the group consisting of pancreatic islet cells, neural cells, renal cortex cells, vascular endothelial cells, thyroid cells, adrenal cells, thymic cells, ovarian cells and hepatic cells.

19. the process of Claim 18, wherein the tissue cells are pancreatic islet cells.

20. The process of claim 19, wherein an effective amount of said coated islets implanted into a diabetic mouse allows said mouse to remain euglycemic for at least 6 months after implantation.

21. The process of Claim 19, wherein said diabetic mouse remains euglycemic for at least 12 months after implantation.

22. The process of Claim 19, wherein said diabetic mouse remains euglycemic for at least 18 months after implantation.

## Patentansprüche

1. Gereinigte nicht-fibrinognene Alginat-Zusammensetzung, die geeignet ist zum Überziehen von funktionellen Gewebe- oder Zelltransplantaten mit einer wesentlich geringeren Menge an Fucose, Polyphenolen und Proteinen,
wobei das nicht-fibrinogene Alginat so gereinigt ist, daß es weniger als 0,02 Gew.% Fucose und weniger als 0,2 Gew.% Polyphenole, angegeben in Werten für Tanninsäure, enthält;
wobei das Molekulargewicht des Alginats im Bereich von 6 bis 120 Kilodalton liegt;
wobei das Alginat ein Gemisch aus Mannuronat und Guluronat umfaßt, wobei die Menge an Mannuronat, berechnet als Verhältnis von Mannuronat/Mannuronat + Guluronat, in dem Alginat im Gewichtsbereich von 0,25 bis 0,75 liegt;
wobei das Alginat nicht-fibrinogen ist, wenn es zum Überziehen von Zellen verendet wird, und
wobei die mit dem nicht-fibrinogenen Alginat überzogenen Zellen, wenn sie einer Maus implantiert werden, während mindestens 60 Tagen nach der Implantation keine Fibrose hervorrufen.

2. Zusammensetzung nach Anspruch, hergestellt nach einem Reinigungsverfahren, umfassend die folgenden Stufen:
(a) Herstellen einer wäßrigein Alginatlösung, umfassend ein Gemisch aus Mannuronat/Mannuronat + Guluronat in einem Gewichtsverhältnis von 0,25 zu 0,75 und ein Chelatierungsmittel für zweiwertige Metallionen;
(b) Zusammenbringen der Alginatlösung mit gebleichter Aktivkohle in einer Menge und während einer Zeit, die ausreichen, um Fucose, Polyphenole, Proteine und andere Verunreinigungen, die in dem Alginat vorhanden sind, zu absorbieren, um ein nicht-fibrinogenes Alginat herzustellen, und anschließendes Entfernen der Aktivkohle von der Alginatlösung;
(c) Zugeben von Ethanol zu der Alginatlösung in einer Menge, die wirksam ist, um das Alginat aus der Alginatlösung auszufällen, und
(d) Isolieren der ausgefällten nicht-fibrinognen Alginat-Zusammensetzung.

3. Zusammensetzung nach Anspruch 2, wobei die ausgefällte nicht-fibrinogne Alginat-Zusammensetzung der Stufe (d) verwendet wird, um ein lebensfähiges funktionelles Gewebe oder Zellen zu überziehen, und mit zweiwertigen Metallionen zu einem Gel geformt wird.

4. Zusammensetzung nach Anspruch 3, wobei die in der Zusammensetzung verbliebene Menge an Fucose kleiner ist als 0,01 Gew.%.

5. Zusammensetzung nach Anspruch 3, wobei die in der Zusammensetzung verbliebene Menge an Fucose kleiner ist als 0,005 Gew.%.

6. Zusammensetzung nach Anspruch 3, wobei die in der Zusammensetzung verbliebene Mange an Polyphenol kleiner ist als 0,1 Gew.%, in Werten für Tanninsäure.

7. Zusammensetzung nach Anspruch 3, wobei die in der Zusammensetzung verbliebene Mange an Polyphenol kleiner ist als 0,075 Gew.%, in Werten für Tanninsäure.

8. Zusammensetzung nach Anspruch 2, wobei in Stufe (b) das Verhältnis von Alginat zu Aktivkohle von 1:1 bis 1:20 beträgt.

9. Nicht-fibrinogenes Gel aus zweiwertigen Metallionen und Alginat, umfassend zweiwertige Metallionen und ein nicht-fibrinogenes Alginat, das gereinigt worden ist mit Aktivkohle mit großer Oberfläche und das ein Molekulargewicht von 6 bis 120 kD, ein Gewichtsverhältnis von Mannuronat/(Mannuronat + Guluronat) von 0,25 bis 0,75 aufweist, wobei die Menge an Fucose kleiner als 0,02 Gew.% ist und die Menge an Polyphenol, in Werten für Tanninsäure, kleiner als 0,2 Gew.% ist und wobei eine mit dem Alginatgel überzogene Zelle, wenn sie einer BALBIC-Maus implantiert wird, während mindestens 60 Tagen nach der Implantation im wesentlichen keine Fibrose hervorruft.

10. Alginatgel nach Anspruch 9, wobei das Gel während mindestens 6 Monaten nach der Implantation im wesentlichen keine Fibrose hervorruft.

11. Alginatgel nach Anspruch 9, wobei das Gel während mindestens 12 Monaten nach der Implantation im wesentlichen keine Fibrose hervorruft.

12. Alginatgel nach Anspruch 9, wobei das Gel während mindestens 18 Monaten nach der Implantation im wesentlichen keine Fibrose hervorruft.

13. Verfahren zum Überziehen von Gewebe mit einem nicht-fibrinogenen Alginat, umfassend:
(a) Herstellen einer wäßrigen Alginatlösung, umfassend ein Alginat mit einem Molekulargewicht von 6 bis 120 kD, umfassend ein Gemisch aus Mannuronat/Mannuronat + Guluronat in einem Gewichtsverhältnis von 0,25 zu 0,75 und einem Chelatierungsmittel für zweiwertige Metallionen;
(b) Zusammenbringen der Alginatlösung mit gebleichter Aktivkohle in einer Menge und während einer Zeit, die ausreichen, um in dem Alginat vorhandene Fucane zu absorbieren, um ein nicht-fibrinogenes Alginat mit einer wesentlich geringeren Menge an Fucose, Polyphenolen und Proteinen herzustellen, wobei die Menge an Fucose auf weniger als 0,02 Gew.% und die Menge an Polyphenolen, angegeben in Werten für Tanninsäure, auf weniger als 0,2 Gew.% verringert ist;
(c) Entfemen der gebleichten Aktivkohle von der Alginatlösung:
(d) Zugeben von Ethanol zu der Alginatlösung in einer Menge, die wirksam ist, um das Alginat aus der Alginatlösung auszufällen,
(e) Isolieren des ausgefällten Alginats und
(f) Überziehen von lebensfähigem physiologisch aktivem Gewebe mit dem ausgefällten nicht-fibrinogenen Alginat.

14. Verfahren nach Anspruch 13, wobei die gebleichte Aktivkohle erhalten worden ist durch Zusammenbringen von Aktivkohle mit einer Teilchengröße von 100 mesh oder kleiner mit einer 0,005 bis 0,50 M Natriumhypochlorit-Lösung während etwa 5 bis 30 Minuten.

15. Verfahren nach Anspruch 14, wobei das Verhältnis von gebleichter Aktivkohle zu Alginat in der Alginatlösung 1:1 bis 1:20 (Gew./Gew.) beträgt.

16. Verfahren nach Anspruch 15, wobei das nicht-fibrinogene Alginat zur Herstellung einer Alginatgel-Kapsel, umfassend lebensfähiges physiologisch aktives überzogenes Gewebe oder Zellen, verwendet wird.

17. Verfahren nach Anspruch 16, wobei der Überzug eine Dicke von mindestens 10 um und weniger als 200 µm hat..

18. Verfahren nach Anspruch 16, wobei das Gewebe mindestens eine oder mehrere Zellarten, ausgewählt aus der Gruppe, bestehend aus Pankreasinselzellen, Nervenzellen, Nierenrindenzellen, Gefäßendothelzellen, Schilddrüsenzellen, Nebennierenzellen, Thymuszellen, Ovarzellen und Leberzellen, umfaßt.

19. Verfahren nach Anspruch 18, wobei die Gewebezellen Pankreasinselzellen sind.

20. Verfahren nach Anspruch 19, wobei eine wirksame Menge der überzogenen Inseln, wenn sie einer diabetischen Maus implantiert werden, ermöglicht, daß die Maus mindestens 6 Monate nach der Implantation euglykämisch bleibt.

21. Verfahren nach Anspruch 19, wobei die diabetische Maus mindestens 12 Monate nach der Implantation euglykämisch bleibt.

22. Verfahren nach Anspruch 19, wobei die diabetische Maus mindestens 18 Monate nach der Implantation euglykämisch bleibt.

## Revendications

1. Composition d'alginate non-fibrogène purifié appropriée pour le revêtement de transplants de tissus ou de cellules fonctionnels, ayant une quantité substantiellement réduite de fucose, de polyphénols et de protéines,
dans laquelle l'alginate non-fibrogène est purifié pour contenir moins de 0,02 % en poids de fucose et moins de 0,2 % en poids de polyphénols, exprimés en termes d'acide tannique;
dans laquelle la masse moléculaire de l'alginate est dans l'intervalle de 6 à 120 kilodaltons;
dans laquelle l'alginate comprend un mélange de mannuronate et de guluronate dans lequel la quantité de mannuronate, calculée en tant que rapport de mannuronate/mannuronate + guluronate, dans l'alginate est dans l'intervalle en poids de 0,25 à 0,75;
dans laquelle l'alginate est non-fibrogène lorsqu'il est utilisé pour le revêtement de cellules; et
dans laquelle lesdites cellules revêtues avec ledit alginate non-fibrogène implantées dans une souris n'induisent substantiellement pas de fibrose sur au moins 60 jours après l'implantation.

2. Composition selon la revendication 1, préparée par un procédé de purification, et comprenant les étapes de:
(a) préparation d'une solution aqueuse d'alginate comprenant un mélange de mannuronate/mannuronate + guluronate dans un rapport en poids de 0,25 à 0,75 et un agent chélatant des ions métalliques divalents;
(b) mise en contact de ladite solution d'alginate avec du carbone activé décoloré dans une quantité et pendant une durée suffisantes pour que le fucose, les polyphénols, les protéines et d'autres contaminants présents dans ledit alginate soient adsorbés pour produire un alginate non-fibrogène et ensuite séparation du dit carbone activé de ladite solution d'alginate; et
(c) addition d'éthanol à ladite solution d'alginate dans une quantité efficace pour la précipitation du dit alginate à partir de ladite solution d'alginate; et
(d) isolement de ladite composition d'alginate non-fibrogène, précipitée.

3. Composition selon la revendication 2, dans laquelle la composition d'alginate non-fibrinogène précipitée de l'étape (d) est utilisée pour le revêtement d'un tissu ou de cellules fonctionnels, viables, et gélifiée avec des ions de métal divalent.

4. Composition selon la revendication 3, dans laquelle la quantité de fucose restant dans la composition est inférieure à 0,01 % en poids.

5. Composition selon la revendication 3, dans laquelle la quantité de fucose restant dans la composition est inférieure à 0,005 % en poids.

6. Composition selon la revendication 3, dans laquelle la quantité de polyphénol restant dans la composition est inférieure à 0,1 % en poids en termes d'acide tannique.

7. Composition selon la revendication 3, dans laquelle la quantité de polyphénol restant dans la composition est inférieure à 0,075 % en poids en termes d'acide tannique.

8. Composition selon la revendication 2, dans laquelle dans l'étape (b) le rapport de l'alginate au carbone activé est de 1:1 à 1:20.

9. Gel d'ion métallique divalent-alginate non-fibrogène, comprenant des ions de métal divalent et un alginate non-fibrogène purifié avec du carbone activé à surface importante, ayant une masse moléculaire de 6 à 120 kD, un rapport en poids de mannuronate/(mannuronate + guluronate) de 0,25 à 0,75, dans lequel la quantité de fucose est inférieure à 0,02 % en poids et la quantité de polyphénol est inférieure à 0,2 % en poids en termes d'acide tannique, et tandis que, lorsqu'une cellule revêtue avec ledit gel d'alginate est implantée dans une souris BALB/C, il n'induit substantiellement pas de fibrose pendant au moins 60 jours après implantation.

10. Gel d'alginate selon la revendication 9, dans lequel ledit gel ne provoque substantiellement pas de fibrose pendant au moins 6 mois après implantation.

11. Gel d'alginate selon la revendication 9, dans lequel ledit gel ne provoque substantiellement pas de fibrose pendant au moins 12 mois après implantation

12. Gel d'alginate selon la revendication 9, dans lequel ledit gel ne provoque substantiellement pas de fibrose pendant au moins 18 mois après implantation.

13. Procédé pour le revêtement de tissu avec un alginate non-fibrogène, comprenant:
(a) la préparation d'une solution aqueuse d'alginate comprenant un alginate ayant une masse moléculaire de 6 à 120 kD, comprenant un mélange de mannuronate/mannuronate + guluronate dans un rapport en poids de 0,25 à 0,75 et un agent chélatant des ions métalliques divalents;
(b) la mise en contact de ladite solution d'alginate avec du carbone activé décoloré dans une quantité et pendant une durée suffisantes pour que les fucanes présents dans ledit alginate soient adsorbés pour produire un alginate non-fibrogène ayant une quantité substantiellement réduite de fucose, de polyphénols et de protéines, tandis que la quantité de fucose est réduite à moins de 0,02 % en poids et la quantité de polyphénol, exprimée en termes d'acide tannique, à moins de 0,2 % en poids;
(c) la séparation du dit carbone activé décoloré de ladite solution d'alginate;
(d) l'addition d'éthanol à ladite solution d'alginate dans une quantité efficace pour la précipitation du dit alginate à partir de ladite solution d'alginate;
(e) l'isolement du dite alginate précipité; et
(f) le revêtement de tissu physiologiquement actif, viable, avec ledit alginate non-fibrogène précipité.

14. Procédé selon la revendication 13, dans lequel ledit carbone activé décoloré est obtenu par mise en contact de charbon activé ayant une taille de particules de 100 mesh ou moins avec une solution d'hypochlorite de sodium 0,005 à 0,50 M pendant environ 5 à 30 minutes.

15. Procédé selon la revendication 14, dans lequel le rapport du carbone activé décoloré à l'alginate dans ladite solution d'alginate est de 1:1 à 1:20 (en poids:poids).

16. Procédé selon la revendication 15, dans lequel l'alginate non-fibrogène est utilisé pour la préparation d'une capsule de gel d'alginate, comprenant du tissu ou des cellules revêtus, physiologiquement actifs, viables.

17. Procédé selon la revendication 16, dans lequel le revêtement a une épaisseur d'au moins 10 µm et inférieure à 200 µm.

18. Procédé selon la revendication 16, dans lequel le tissu comprend au moins un ou plusieurs types de cellules choisis dans le groupe consistant en les cellules d'ilôts pancréatiques, les cellules nerveuses, les cellules du cortex rénal, les cellules endothéliales vasculaires, les cellules thyroïdiennes, les cellules des surrénales, les cellules thymiques, les cellules ovariennes et les cellules hépatiques.

19. Procédé selon la revendication 18, dans lequel les cellules tissulaires sont des cellules d'ilôts pancréatiques.

20. Procédé selon la revendication 19, dans lequel une quantité efficace des dits ilôts revêtus implantés dans une souris diabétique permet à ladite souris de rester euglycémique pendant au moins 6 mois après l'implantation.

21. Procédé selon la revendication 19, dans lequel ladite souris diabétique reste euglycémique pendant au moins 12 mois après l'implantation.

22. Procédé selon la revendication 19, dans lequel ladite souris diabétique reste euglycémique pendant au moins 18 mois après l'implantation.
